# EUROPEAN PATENT SPECIFICATION

(11) **EP 0 815 831 B1**
(45) Date of publication and mention of the grant of the patent: **03.09.2003**
(21) Application number: 96904999.8
(22) Date of filing: 06.03.1996
(51) Int. Cl.: A61K 7/16

(54) **DENTIFRICE COMPOSITION**
ZAHNPFLEGEMITTEL
COMPOSITION DENTIFRICE

(30) Priority: 10.03.1995 JP 5078495; 01.03.1996 JP 6892396
(43) Date of publication of application: 07.01.1998
(73) Proprietor: Eisai Co., Ltd., Tokyo 112-0002 (JP); Ventrep Sante, Inc., Machida-shi, Tokyo 194 (JP)
(72) Inventor: TAKEDA, Yoshinori, Nagareyama-shi, Chiba 270-01 (JP); IMANISHI, Syogo, Machida-shi, Tokyo 194 (JP)
(74) Representative: Hansen, Bernd, Dr. Dipl.-Chem.
(86) International application number: JP9600528
(87) International publication number: WO96028134

(56) References cited:
- FR-A- 2 124 311
- JP-A- 3 127 718
- JP-A- 4 082 821
- JP-A- 5 331 019
- JP-A- 59 096 199
- JP-A- 60 239 408
- JP-A- 62 016 506

## Description

### TECHNICAL FIELD

The present invention relates to dentifrice compositions which can impart good gloss to teeth.

### BACKGROUND ART

Dentifrice compositions have effects of cleaning oral cavities, preventing various diseases, and so on. Many parsons brush their teeth once to several times a day. It is also important as etiquette or a symbol of smiles to maintain one's teeth white. Therefore, various means have been exerted on commercially available dentifrice compositions for the purpose of whitening teeth.

The conventional dentifrice compositions are useful in cleaning oral cavities and can keep teeth their inherent whiteness. However, they cannot impart higher gloss or brightness than the whiteness inherent in teeth to the teeth.

Japanese Patent Application Laid-Open No. 82821/1992 discloses an example where shellac is used as a a beautifying agent for teeth. However, this beautifying agent has involved such problems that since it is used in the form of an ethanol solution, oral mucosa is given a stronger stimulus. There is therefore a demand for development of a product which is easy to use and can impart good gloss to teeth with certainty.

### DISCLOSURE OF THE INVENTION

The present inventors have carried out an extensive investigation with a view toward solving the above-described problems. As a result, it has been found that the problems can be solved by the following means, thus. leading to completion of the present invention.

### BEST MODE FOR CARRYING OUT THE INVENTION

The present invention is directed to a dentifrice composition for imparting good gloss to teeth, comprising shellac. The present invention is also directed to a dentifrice composition for imparting good gloss to teeth, comprising shellac and further a disinfectant.

The present invention is still further directed to a dentifrice composition in the form of gel, paste, powder, granules or liquid, comprising wax or shellac, or a dentifrice composition in the form of gel, paste, powder, granules or liquid, including wax or shellac and further a fluorinated oil and/or a disinfectant.

The shellac (Japanese pharmacopoeia) useful in the practice of the present invention is a resinous substance obtained by purifying a secretion which covers the body of a lac insect [*Laccifer Lacca Kerr (Coccidae* )].
The shellac is a scaly fine piece of pale-yellowish brown or brown, is hard, brittle and glossy and gives no odor or a slight specific odor. It is insoluble in water, ether, hexane and isooctane, but soluble in ethanol and propylene glycol. For example, the solubility of the shellac in propylene glycol is 100 mg/ml at 25°C and 500 mg/ml at 37°C . The shellac generally contains 5-6% of wax. However, purified shellac is a dewaxed substance. The main component of the shellac is a mixed lactide composed mainly of 2 mol of aleuric acid, 2 mol of Shelloic acid and 4 mol of shellac resin acid. The probable structural formula thereof is said to be represented by the following formula:

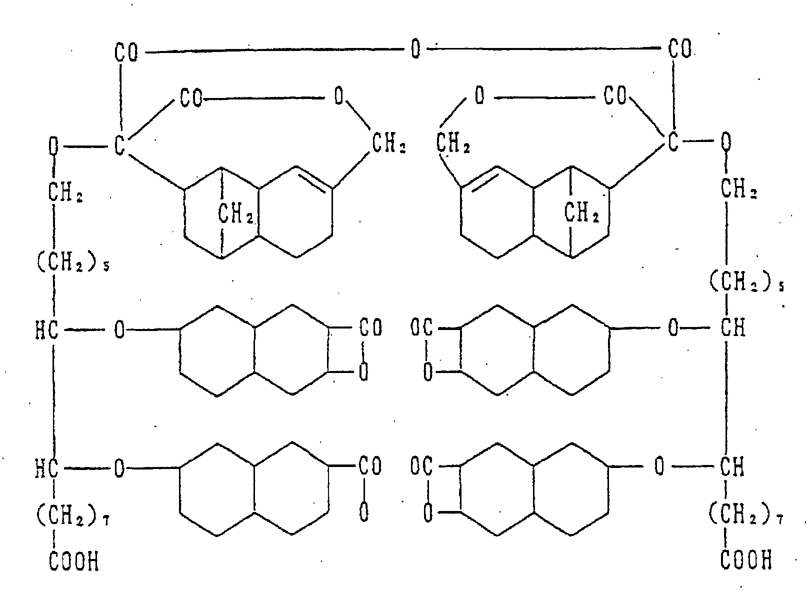

In the present invention, a fluorinated oil may also be contained in the dentifrice compositions. The fluorinated oil means perfluoro(polyether), and is available under a name of, for example, FOMBLIN HC (Trade Mark). FOMBLIN HC (Trade Mark) includes various grades. However, the fluorinated oil may be suitably selected for use from among the various grades of FOMBLIN HC (Trade Mark). The combined use of the fluorinated oil can more enhance the gloss of teeth.

In the present invention, a disinfectant may be further contained in the dentifrice compositions. Specific examples of the disinfectant may include chlorhexidine hydrochloride, isopropylmethylphenol, cetylpyridinium chloride, dequalinium chloride, benzalkonium chloride, benzethonium chloride, alkyldiaminoethylglycine hydrochlorides and triclosan.

The content of shellac used in the dentifrice compositions according to the present invention is generally 0.01% to 10%, preferably 0.1% to 7%, more preferably 1% to 5%, based on the total weight of each of the composition.

The content of the fluorinated oil is generally 0.01% to 2%, preferably 0.05% to 1%, more preferably 0.1% to 0.5%, based on the total weight of the composition.

The content of the disinfectant is generally 0.001% to 1%, preferably 0.005% to 0.5%, more preferably 0.01% to 0.2%, based on the total weight of the composition.

No particular limitation is imposed on the form of a dentifrice in the present invention. However, dentifrices in the form of, for example, gel, paste, powder, granules or liquid is preferred.

In the present invention, it is most preferable to mix the shellac with the conventional dentifrice compositions. Substances used in the conventional dentifrice compositions may include polishing agents such as heavy calcium carbonate and aluminum hydroxide, wetting agents such as propylene glycol and sorbit solutions, foaming agents such as sodium lauryl sulfate, thickening agents such as carrageenan and sodium carboxymethyl cellulose, perfume bases, flavors such as sodium saccharin, preservatives such as parabens, and various kinds of medicinally-effective ingredients. Although the wax is insoluble in water, it is easily soluble in propylene glycol, which is a component in the dentifrice compositions, or the like. Therefore, the wax is convenient for use.

A process for producing the dentifrice compositions according to the present invention is the same as the production process for the conventional dentifrice compositions except that the shellac is dissolved in a substance commonly used in the conventional dentifrice compositions, such as propylene glycol. FOMBLIN HC (Trade Mark) is insoluble in almost all solvents, but may be mixed for use with, for example, propylene glycol or glycerol because its emulsifiability and dispersibility are good. The disinfectant may also be dissolved or dispersed for use in a solvent generally used in dentifrice compositions.

No particular limitation is imposed on the usage of the dentifrice compositions according to the present invention. However, they may be generally used in the same manner as in the conventional dentifrice compositions upon brushing in the morning and the evening and before sleeping. When the dentifrice composition according to the present invention is liquid, it may be coated directly on teeth with a brush or the like.

### EXAMPLES

The present invention will hereinafter be described in more detail by the following Examples. However, the present invention is not limited to these examples.

### Example 1: Toothpaste

| Components | wt.% |
|---|---|
| Calcium carbonate | 36.0 |
| Sorbitol (70%) | 15.0 |
| Glycerol | 5.0 |
| Propylene glycol | 7.0 |
| Shellac | 2.0 |
| Sodium carboxymethyl cellulose | 1.0 |
| Sodium lauryl sulfate | 1.3 |
| Flavor | 1.0 |
| Sodium saccharin | 0.1 |
| Propyl p-hydroxybenzoate | 0.1 |
| Sodium benzoate | 0.1 |
| Purified water | 31.4 |
| Total | 100.0 |

Shellac was dissolved under heat in propylene glycol in advance. Separately from the above, sodium carboxymethyl cellulose was fully dispersed in glycerol. Propyl p-hydroxybenzoate was dissolved in the flavor. On the other hand, sorbitol, sodium saccharin, sodium benzoate and purified water were charged into a mixer equipped with a deaerator, thereby homogeneously mixing them into a solution. The solution of shellac in propylene glycol and the dispersion of carboxymethyl cellulose in glycerol were added to the above-prepared solution to mix them, thereby preparing a uniform viscous liquid. After air contained in the viscous liquid was then deaerated, stirring was stopped once, calcium carbonate and sodium lauryl sulfate were added, and the resultant mixture was stirred again under a condition of reduced pressure to prepare uniform toothpaste. Finally, the flavor was added and mixed into the paste to produce a dentifrice composition according to the present invention.

### Example 2: Toothpaste

| Components | wt.% |
|---|---|
| Calcium phosphate | 40.0 |
| Silicic acid anhydride | 2.0 |
| Glycerol | 13.0 |
| Propylene glycol | 10.0 |
| Shellac | 3.0 |
| Carrageenan | 0.9 |
| Locust bean gum | 0.2 |
| Sodium lauryl sulfate | 1.0 |
| Flavor | 1.0 |
| Sodium saccharin | 0.1 |
| Propyl p-hydroxybenzoate | 0.1 |
| Sodium benzoate | 0.1 |
| Purified water | 28.6 |
| Total | 100.0 |

The above components were mixed into a solution in the same manner as in Example 1 to obtain a dentifrice composition according to the present invention.

### Example 3: Toothpaste

| Components | wt.% |
|---|---|
| Silicic acid anhydride | 15.0 |
| Sorbitol (70%) | 65.0 |
| Glycerol | 3.0 |
| Propylene glycol | 8.0 |
| Shellac | 2.0 |
| Sodium carboxymethyl cellulose | 0.8 |
| Sodium lauryl sulfate | 1.5 |
| Flavor | 1.3 |
| Sodium saccharin | 0.1 |
| Methyl p-hydroxybenzoate | 0.1 |
| Sodium benzoate | 0.1 |
| Purified water | 3.1 |
| Total | 100.0 |

The above components were mixed into a solution in the same manner as in Example 1 to obtain a dentifrice composition according to the present invention.

An experiment for judging glossiness on surfaces of teeth was then conducted. More specifically, teeth of 16 volunteers (9 men and 7 women) were brushed with a toothpaste (A) according to the present invention and a control toothpaste (B) with no shellac incorporated therein, whereby the glossiness of the teeth tested were visually judged by 3 judges experienced in the clinical judgment for at least 10 years.

### Experimental method:

Teeth to be tested were determined as surfaces of maxillomandibular anterior teeth on the side of lips. Right after the three judges visually observed the state of the surfaces of the teeth to be tested, one of the test toothpastes was taken on a toothbrush which is usually used by the volunteers by about 2 cm to freely brush only the surfaces of the test teeth for about 1 minute in such a manner that the volunteers do not let the judges know which toothpaste they used. However, the numbers of the volunteers who used the respective toothpastes were the same (8 persons). After completion of the brushing, the state of gloss on the surfaces of the teeth tested was judged by the three judges. Criteria for the judgment were 4 ranks that the gloss of the surfaces of the teeth tested was "unchanged", "a toss-up between changed and unchanged", "somewhat changed better" and "apparently changed better" after the brushing compared with the gloss before the brushing.

### Results:

The results of the judgment are shown below.

| | Invention toothpaste (A) | Control toothpaste (B) |
|---|---|---|
| Unchanged | 3 | 9 |
| Toss-up | 5 | 7 |
| Somewhat changed better | 13 | 8 |
| Apparently changed better | 3 | 0 |

As a result of a Mann-Whitney test, a statistically significant difference was recognized at a significance level of 1%, between the group in which the invention toothpaste (A) was used and the group in which the control toothpaste (B) was used.

### Conclusion:

The surfaces of the teeth after brushed with the toothpaste (A) according to the present invention was judged to be more glossy compared with those after brushed with the control toothpaste (B).

### INDUSTRIAL APPLICABILITY

When the dentifrice compositions according to the present invention are used, the whiteness inherent in teeth can be given to the teeth, and good gloss, brightness and luster can be further imparted to the teeth. Since teeth have numberless flaws, their brightness is lessened. When the dentifrice composition according to the present invention is used, however, the shellac contained in the composition covers the surfaces of the teeth, thereby smoothing them.
Therefore, it is considered that irregular reflection of light is prevented, and so, good gloss, brightness and luster can be imparted to the teeth.

In the present invention, a fluorinated oil and/or a disinfectant may be further contained in the dentifrice compositions, whereby the gloss and the like of the teeth can be further enhanced, and propagation of bacteria in the teeth covered can be prevented. Namely, the fluorinated oil has an action that gloss can be enhanced.

On the other hand, when the teeth are covered with the wax or shellac, anaerobic bacteria may propagate in the teeth in some cases due to low air permeability of the shellac film. However, this propagation can be prevented by containing the disinfectant in the dentifrice compositions.

## Claims

1. A dentifrice composition for imparting good gloss to teeth, comprising shellac in an amount of 0,01% to 10% based on the total weight of the composition.

2. A dentifrice composition according to claim 1 which further comprises a disinfectant.

3. The dentifrice composition according to claim 2, wherein the disinfectant is chlorhexidine hydrochloride, isopropylmethylphenol, cetylpyridinium chloride, dequalinium chloride, benzalkonium chloride, benzethonium chloride, an alkyldiaminoethylglycine hydrochloride or triclosan.

4. The dentifrice composition according to any one of claim 1 to 3, wherein the dentifrice is in the form of gel, paste, powder, granules or liquid.

## Patentansprüche

1. Zahnpasta-Zusammensetzung, um den Zähnen einen guten Glanz zu verleihen, umfassend Schellak in einer Menge von 0,01 bis 10 %, bezogen auf das Gesamtgewicht der Zusammensetzung.

2. Zahnpasta-Zusammensetzung gemäss Anspruch 1, die ausserdem ein Desinfektionsmittel umfasst.

3. Zahnpasta-Zusammensetzung gemäss Anspruch 2, wobei das Desinfektionsmittel Chlorhexidin-hydrochlorid, Isopropylmethylphenol, Cetylpyridiniumchlorid, Dequaliniumchlorid, Benzalkoniumchlorid, Benzethoniumchlorid, ein Alkyldiaminoethylglycinhydrochlorid oder Triclosan ist.

4. Zahnpasta-Zusammensetzung gemäss einem der Ansprüche 1 bis 3, wobei die Zahnpasta in Form eines Gels, einer Paste, eines Pulvers, Körnern oder einer Flüssigkeit vorliegt.

## Revendications

1. Composition de dentifrice afin de transmettre une bonne brillance aux dents, comprenant de la gomme-laque en une quantité de 0,01 % à 10 % sur la base du poids total de la composition.

2. Composition de dentifrice selon la revendication 1, qui comprend en outre un désinfectent.

3. Composition de dentifrice selon la revendication 2, dans laquelle le désinfectant est un chlorhydrate de chlorhexidine, un isopropylméthylphénql, un chlorure de cétylpyridinium, un chlorure de déqualinium, un chlorure de benzalkonium, un chlorure de benzéthonium, un chlorhydrate d'alkyldiaminoéthylglycine ou un triclosane.

4. Composition de dentifrice selon l'une quelconque des revendications 1 à 3, dans laquelle le dentifrice est sous la forme d'un gel, d'une pâte, d'une poudre, de granulés ou liquide.
